# EUROPÄISCHE PATENTSCHRIFT

(11) **EP 1 073 445 B1**
(45) Veröffentlichungstag und Bekanntmachung des Hinweises auf die Patenterteilung: **23.07.2003**
(21) Anmeldenummer: 99916713.3
(22) Anmeldetag: 22.04.1999
(51) Int. Cl.: A61K 35/78, A61P 17/02

(54) **STOFFGEMISCH ZUR TOPISCHEN ANWENDUNG ENTHALTEND OLIVENÖL UND HONIG**
SUBSTANCE MIXTURE FOR TOPICAL APPLICATION COMPRISING OLIVE OIL AND HONEY
MELANGE DE SUBSTANCES POUR APPLICATION TOPIQUE COMPRENANT DU MIEL ET DE L'HUILE D'OLIVE

(30) Priorität: 24.04.1998 AT 69098
(43) Veröffentlichungstag der Anmeldung: 07.02.2001
(73) Patentinhaber: Dado, Suleiman, 1030 Wien (AT)
(72) Erfinder: Dado, Suleiman, 1030 Wien (AT)
(86) Internationale Anmeldenummer: AT9900098
(87) Internationale Veröffentlichungsnummer: WO99055349

(56) Entgegenhaltungen:
- WO-A-97/42963
- DE-A- 2 123 525
- FR-A- 2 651 123
- FR-A- 2 729 860
- GB-A- 2 228 411
- DATABASE WPI Section Ch, Week 9818 Derwent Publications Ltd., London, GB; Class D13, AN 98-205068 XP002108945 & MX 9 503 503 A (CERVANTES SAN JUAN A) , 1. Februar 1997
- PATENT ABSTRACTS OF JAPAN vol. 017, no. 158 (C-1041), 29. März 1993 & JP 04 321617 A (HARUO KATAYAMA), 11. November 1992
- DATABASE WPI Section Ch, Week 9633 Derwent Publications Ltd., London, GB; Class B04, AN 96-323079 XP002108946 & ES 2 087 036 A (CABALLERO AGUERRI I) , 1. Juli 1996
- DATABASE WPI Section Ch, Week 9612 Derwent Publications Ltd., London, GB; Class B04, AN 96-107601 XP002044773 & ES 2 080 697 A (SORIA NATURAL SA) , 1. Februar 1996
- DATABASE WPI Section Ch, Week 9849 Derwent Publications Ltd., London, GB; Class A96, AN 98-581232 XP002108947 & RU 2 110 251 C (BIOCOSMETIC WKS CO LTD) , 10. Mai 1998

## Beschreibung

Die Erfindung betrifft die Verwendung eines Stoffgemischs umfassend Honig und Olivenöl.

Mittel, die derzeit zur Behandlung von Hämorrhoiden (knotenförmige Erweiterung der Äste der Arteria oder Vena rectalis sup. im Bereich der arteriell und venös durchbluteten Corpora cavernosa recti) verwendet werden, enthalten sehr oft Kortikoide, deren Verwendung aufgrund der starken Nebenwirkungen aber mit großen Schwierigkeiten und Risiken verbunden ist. Es gibt nun auch einige kortikoidfreie Arzneimittel, wie z.B. Acetonal-Hämorrhoidal®, Hädensa®, Mucotherm-Zäpfchen®, Sperti Präparation® oder Sulgan®, um nur einige zu nennen, wobei bei allen eine Überempfindlichkeit gegen einen der Bestandteile dieser Präparate auftreten kann. Bei den beiden Letztgenannten können auch allergische Reaktionen auftreten. Weiters haben diese Mittel keine rasche und sichere Heilung zur Folge, sondern wirken meist nur lindernd.

Aus der MX 9 503503 A ist eine Präparation zur Stärkung von Haar und Kopfhaut bekannt, wobei die Präparation unter anderem Olivenöl und Honig umfasst.

Die GB 2 228 411 A betrifft kosmetische Mischungen, die unter anderem bis zu 5% Honig und 20-40% Olivenöl umfassen.

In der WO 97/42963 A wird eine pharmazeutische Präparation beschrieben, die Olivenöl sowie gelbes Honigwachs umfasst, und zwar in den Mengen von etwa 10% Honigwachs und 80-85% Olivenöl.

Die FR 2 729 860 A und der Abstract der ES 2 087 036 A betreffen Präparationen, die lediglich Olivenöl, jedoch keinen Honig umfassen, und die FR 2 651 123 A und die DE 21 23 525 A betreffen Präparationen, die lediglich Honig, jedoch kein Olivenöl umfassen. Diese Präparationen werden zu medizinischen bzw. kosmetischen Zwecken verwendet.

Gemäß der JP 04 321617 A wird eine Gelpräparation für kosmetische und medizinische Zwecke verwendet, die Olivenöl, Honig und Wasser im Verhältnis 4:1:1 umfasst.

Der Abstract der ES 2 080 697 A betrifft eine dermatologische Creme umfassend 45 1 Olivenöl, Propolis und 14 kg Wachs.

In der RU 2 110 251 C wird eine Handcreme beschrieben, die unter anderem 1-15% Olivenöl und 4-14% Bienenwachs und Honig umfasst.

Hämorrhoidale Erkrankungen weisen spezifische Eigenschaften auf und sind in der Regel anders zu behandeln als Wunden, Ekzeme, etc. an anderen Körperstellen.

Es ist daher Aufgabe der vorliegenden Erfindung, ein Stoffgemisch zur Verfügung zu stellen, welches zur Herstellung eines Mittels zur Behandlung von hämorrhoidalen Erkrankungen geeignet ist, welches aber auch aus möglichst naturnahen komplexen Komponenten zusammengesetzt ist und dadurch gut verträglich und ohne negative Nebenwirkungen ist.

Diese Aufgaben werden erfindungsgemäß gelöst durch die Verwendung eines Stoffgemisches, umfassend Honig und Olivenöl zur Herstellung eines Mittels zur Behandlung von hämorrhoidalen Erkrankungen. Mit der erfindungsgemäßen Verwendung können die an sich bekannten heilenden Wirkungen von Honig und Olivenöl ausgenützt werden, wobei sich die therapeutischen Effekte bei dieser Anwendung in synergistischer Weise verstärken.

Durch ein Auftragen auf die zu behandelnde Haut können die Inhaltsstoffe ihre Wirkung direkt an der gewünschten Stelle entfalten und rasch zu sicheren Heilerfolgen führen. Dadurch, dass das erfindungsgemäße Stoffgemisch rein natürliche Substanzen beinhaltet, erfolgt die Anwendung im Gegensatz zu den künstlich hergestellten Arzneimitteln, die chemisch hergestellte Stoffe oft in unnatürlich hohen Konzentrationen aufweisen und deren Anwendung nicht ungefährlich ist, ohne Gegenanzeigen und schädliche Nebenwirkungen.

Die Behandlung beruht dabei auf der Wirkung von rein natürlichen Substanzen, wodurch die Gefahr von Gegenreaktionen jeglicher Art, z.B. Allergien, Überempfindlichkeit gegen Inhaltsstoffe und dergleichen, stark herabgesetzt wird. Des Weiteren soll die hämorrhoidale Erkrankung mit Hilfe des erfindungsgemäßen Stoffgemischs rasch geheilt und nicht nur gelindert werden, wie dies bei den derzeitigen Arzneimitteln oft der Fall ist.

Honig und Olivenöl sind zwar alleine oder in Form von Stoffgemischen zusammen mit anderen Substanzen in der Heilkunde bereits bekannt, jedoch ist bisher noch nie ein Gemisch aus Honig und Olivenöl zur Behandlung von hämorrhoidalen Erkrankungen eingesetzt worden.

Honig wird in der Heilkunst aufgrund seiner unterschiedlichen und ausgeweiteten heilenden und lindernden Wirkungen schon seit Jahrhunderten verwendet.

Es gibt eine Vielzahl von verschiedenen Honigsorten, die, je nach Inhaltsstoffen, unterschiedliche Eigenschaften bezüglich ihrer Anwendung im medizinischen Bereich aufweisen. Der Hauptbestandteil des Honigs ist Invertzucker, weitere Zucker sind Rohrzucker, Maltose und, je nach beflogener Pflanzengattung, aus diesen stammende seltenere Zucker. Neben Zucker beinhaltet Honig weiters Enzyme, wie Invertase, Diastasen, Katalase, Amylase, Phosphatase, Glucoseoxidase, die unter Mitwirkung des Luftsauerstoffs Traubenzucker in Gluconsäure und Wasserstoffperoxid überführt. Dieses stellt Sauerstoff in äußerst reaktiver Form zur Verfügung, der wiederum ein hervorragender Keimtöter und Haltbarmacher ist. Einer der wichtigsten Bestandteile von Honig ist Pollen. An organischen Säuren kommen Apfelsäure, Bernsteinsäure, Gluconsäure, Essigsäure, Ameisensäure, an anorganischen Säuren Phosphorsäure und Salzsäure vor. Honig enthält weiters Mineralstoffe mit einem Anteil von bis zu 3%, darunter Fe, Cu, P, S, K, Na, Mg, Ca, Si, Mn, Cl, Zn, in geringen Mengen sind auch Vitamine (B1, B2, B6, Pantothensäure, Nikotinsäure, H, Folsäure und wenig Vitamin C) und in sehr geringem Maße nahezu alle Aminosäuren enthalten. Des Weiteren wurden Hormone, Azetylcholin, das bei der Nervenreizleitung eine Rolle spielt, Inhibine (Bakterizide) und pflanzliche Farbstoffe, wie Flavone oder Karotine und Aromastoffe (Alkohole, Aldehyde, Ketone und ätherische Öle) gefunden ("Doktor Biene, Bienenprodukte - ihre Heilkraft und Anwendung" von Paul Uccusic, S. 45-53, 2. Auflage, 1987, Wilhelm Heyne Verlag, München).

Honigtauhonige sind reich an Harzen und ätherischen Ölen und kommen daher bei allen Erkrankungen der Atemwege (Bronchialkatarrhe, aber auch zur unterstützenden Behandlung von Lungentuberkulose und Lungenentzündung geeignet), des Urogenitalsystems (Cystitis, Urethriotis, Prostatitis) und als harntreibendes Mittel in Betracht. Weiters kann eine gute Wirkung bei Nierensand und Nierensteinen festgestellt werden. Auch ist eine regulierende Wirkung bei Problemen des Pfortaderkreislaufes (Hämorrhoiden, Pfortaderstauungen, Neigung zu Venentrombosen) postuliert worden. Blütenmischhonige haben eine sehr gute Wirkung auf Allergiker. Lindenhonig wirkt nervenberuhigend und antiseptisch. Melissenhonig wirkt krampflösend und nervenberuhigend. Kastanienhonig wirkt im allgemeinen blutreinigend, bekämpft die Neigung zu Thrombosen, Thrombophlebitis und Krampfadern, um nur einige Beispiele aus der Liste der Honigsorten mit den verschiedenen heilenden Eigenschaften zu nennen ("Doktor Biene", S. 64-71, s.o.). Es werden auch Mischungen von Honig mit anderen pflanzlichen Substanzen beschrieben, die bei den unterschiedlichsten Beschwerden angewendet werden: z.B. eine Paste aus gleichen Teilen Honig, Olivenöl und Propolis gegen Parodontose und Karies; eine Mischung aus 95% Rosenhonig, 5% Borax für Wunden, Furunkeln, Ausschläge und Ekzeme; Propolis in Honig gelöst wirkt vorbeugend gegen Grippe und Schnupfen; Honig und Fenchelsirup im Verhältnis 1:1 hilft gegen Verdauungsbeschwerden, Blähungen, Sodbrennen und Verstopfung. Andere bekannte Mischungen mit heilender Wirkung sind Honig mit Wein, Honig mit Lavendel oder Honig mit Edelkastanien ("Doktor Biene", S. 148-165, s.o.).

Auch Olivenöl wird als Naturheilmittel schon seit langer Zeit gegen verschiedene Beschwerden angewendet. Olivenöl enthält je nach Herkunft und Gewinnung 95 bis 99% Acylglycerole, 0,5 bis 1,5% unverseifbare Anteile und 0,1 bis 3% freie Fettsäuren, Glyceride, Secciridoide und Flavonoide. Die Zusammensetzung der nach Verseifung erhältlichen Fettsäurefraktion variiert je nach Herkunft und Reifegrad der Oliven, mit Ölsäure, Palmitinsäure und Linolsäure als Hauptbestandteile. Weiters kommen phenolische Verbindungen, Kohlenwasserstoffe (hauptsächlich Squalen), Sterole, Triterpenalkohole, Hydroxytriterpensäuren, Tocopherole, Phospholipide, Carotinoide, Chlorophyll und Phaeophytine vor ("Hagars Handbuch der pharmazeutischen Praxis" von R. Hänsel, K. Keller, H. Rimpler, G. Schneider (Hrsg.), Band 5, Drogen E-O, S. 940-945, 5. Auflage, 1993, Springer Verlag).

Nach dem Stand der Technik wird Olivenöl z.B. bei Cholangitis, Cholelithiasis, Ikterus, Flatulenz, Meteorismus, Dysbakterien, Roemheld-Syndrom, bei Obstipation und als Darmgleitmittel angewendet. Außerdem wird Olivenöl bei Gallenwegsleiden, zur Behandlung von Magen- und Darmgeschwüren, bei Nierensteinen sowie in Form einer Emulsion als stickstoffreiche Diät bei Nierenversagen gegeben. Auch zur Wundpflege bei leichten Verbrennungen und bei Psoriasis, zum Erweichen von Krusten bei Ekzemen und als Massagegleitöl in wässriger Emulsion bei Sonnenbrand, als Massageöl zur Rheumabehandlung sowie bei Blutungen wird Olivenöl verwendet ("Hagars Handbuch der pharmazeutischen Praxis" s.o.).

Obwohl, wie erwähnt, Honig und Olivenöl für eine ganze Reihe von Heilzwecken verwendet worden sind, hat sich im Rahmen der vorliegenden Erfindung herausgestellt, dass durch eine Kombination von Honig und Olivenöl in geeignetem Verhältnis diese Stoffe eine ganz besonders heilende Wirkung im Fall von hämorrhoidalen Erkrankungen entfalten, die direkt an der zu behandelnden Stelle rasch eintritt und zu sicheren Erfolgen führt. Wie erwähnt, wird erfindungsgemäß die bekannte Wirkung von Honig gegen Hämorrhoiden, durch die Anwesenheit von Olivenöl derart verstärkt und verbessert, dass Hämorrhoidenleiden nicht nur gelindert, sondern effektiv beseitigt werden können (siehe Beispiel). Im Gegensatz zu den Arzneimitteln, die im Stand der Technik beschrieben sind, wirkt das erfindungsgemäße Stoffgemisch rasch und beseitigt Hämorrhoiden zuverlässig, wie auch in dem Beispiel demonstriert wird.

Es hat sich gezeigt, dass die Verwendung des Stoffgemischs besonders günstig ist, wenn das Gemisch Honig zu 50-90% enthält. Wenn Honig den größeren Anteil des Stoffgemischs ausmacht (mindestens über die Hälfte), so wird dessen regulierende Wirkung bei Problemen des Pfortaderkreislaufes (Hämorrhoiden, Pfortaderstauungen, Neigung zu Venenthrombosen) durch den Zusatz von Olivenöl besonders verstärkt. Wenn Honig etwa 60% des Stoffgemischs ausmacht, scheint die maximale heilende Wirkung gegen diese Beschwerden erreicht zu sein. Dabei ist eine Mischung aus Blütenhonig, Waldhonig und Akazienhonig günstig.

Eine besonders vorteilhafte Wirkung des erfindungsgemäßen Stoffgemischs wird erreicht, wenn das Gemisch Olivenöl zu 5-20% enthält. Optimal ist ein Zusatz von etwa 8,5% Olivenöl.

Es ist vorteilhaft, bei der erfindungsgemäßen Verwendung ein Stoffgemisch vorzusehen, welches zusätzlich eine, mehrere oder alle der folgenden Substanzen enthält: Bienenwachs, Propolis, Kamille, Salbei, Aloe Vera, Thymian, Lavendel und/oder diverse Öle wie Johanneskraut, Weizenöl, u.a..

Bienenwachs wird im kosmetischen und medizinischen Bereich zur Herstellung von Cremes und Salben verwendet, wobei es emulgierend wirkt. Wie Honig, Propolis und Gelee Royale besitzt Bienenwachs therapeutische Eigenschaften und ist daher zur Herstellung von Cremes wesentlich besser geeignet als Wachs mineralischen Ursprungs ("Aromatherapie von A-Z" von Patricia Davis, S. 77, 1998, Knaur-Verlag). Chemisch gesehen bestehen Wachse aus Fettsäuren, die mit höheren Alkoholen verestert sind, Propolis, Farbstoffen und Vitamin A. Seit jeher wird Wachs als Wundpflaster und gegen Hautkrankheiten, zum Einbalsamieren von Leichnamen und in der Geruchs-(Aroma-)therapie verwendet. In der Kosmetik findet Wachs als Bestandteil von Gesichtsmasken oder Enthaarungsmitteln Verwendung ("Doktor Biene", S. 136-139, s.o.).

Propolis besitzt keine konstante, chemische Zusammensetzung. Je nach regionaler Herkunft dieser Substanz besteht sie aus etwa 55% Harz- und Balsamstoffen, etwa 30% Wachs, 5 bis 10% ätherischen Ölen, etwa 2 bis 5% Pollen, Vitaminen und Mikroelementen ("Propolis - Heilkraft aus dem Bienenvolk" von Klaus Nowottnick, S. 26-31, Leopold Stocker Verlag, 1994). Propolis besitzt umfassende und hochwirksame hemmende und abtötende Wirkungen auf zahlreiche Bakterienstämme, fungizide Eigenschaften und wirkt ausgesprochen viruzid. Weiters konnten stark anästhesierende Eigenschaften nachgewiesen werden, es wirkt gegen Verdauungsbeschwerden, Hauterkrankungen und Quetschungen und weist gewebschützende und wundheilende Eigenschaften auf. In verschiedenen Apitherapiekliniken wurden bei Erkrankungen der Herzgefäße und des Kreislaufs durch Kombination von Gelee Royale mit Propolis, Pollen und Kürbiskern überragende Erfolge erzielt. Propolis konnte auch verschiedene Krankheiten im Hals-, Nasen und Ohrenbereich sowie Bronchial- und Lungenleiden kurieren ("Naturheilung mit Honig - Gesundheit aus der Natur" von Arne Lund, S. 34-71, Ludwig Verlag). Viele Ärzte behandeln Krampfadern und periphere Durchblutungsstörungen, periproktische Abszesse, Hämorrhoiden und Analfisteln mit Propolis ("Doktor Biene", S. 110-133, s.o.). Diese Eigenschaften können für das erfindungsgemäße Stoffgemisch optimal ausgenützt werden, wodurch die heilende Wirkung, insbesondere hinsichtlich Durchblutungsstörungen, verstärkt wird.

Kamille (Chamaemelum, Chamomilla) als Arzneimittelzusatz ist in der Medizin weit verbreitet und ist auch für das erfindungsgemäße Stoffgemisch ungemein von Vorteil. Die Inhaltsstoffe der Kamille sind im wesentlichen ätherische Öle, Sesquiterpene, Hydroperoxide, Polyphenole, Polyine, Triterpene und Steroide. Die Wirkung der Kamille ist weitreichend: Antiphlogistisch, muskulotrop spasmolytisch, wundheilungsfördernd, desodorierend, antiseptisch und desinfizierend, hautstoffwechselanregend, um nur einige Eigenschaften zu nennen ("Hagars Handbuch der pharmazeutischen Industrie" von R. Hänsel, K. Keller, H. Rimpler, G. Schneider, Band 4, Drogen A-D, S. 807-830, 5. Auflage, 1992, Springer Verlag). Durch den Zusatz von Kamille zum erfindungsgemäßen Stoffgemisch wirkt dieses weiters schmerz- und krampflindernd, entzündungshemmend, weiters wird die Behandlung von Hämorrhoiden gefördert.

Salbei (Salvia) enthält ätherische Öle, Hydroxyzimtsäuren, Depside, Flavonoide, Di- und Triterpene und Spurenelemente. Salbei besitzt unter anderem antimikrobielle, antivirale, antihidrotische, krampflösend, reizhemmend, antihypertensive, choleretische, spasmolytische, zentrale und antidiabetische Wirkung ("Hagars Handbuch der pharmazeutischen Praxis" von R. Hänsel, K. Keller, H. Rimpler, G. Schneider, Band 6, Drogen P-Z, S. 538-567, 50. Auflage, 1994, Springer Verlag). Aufgrund dieser vielfältigen Eigenschaften wird Salbei in einer bevorzugten Ausführungsform dem Stoffgemisch zugesetzt, wodurch es verstärkt entzündungshemmend und wundheilend wirkt.

Aloe Vera ist der eingedickte Saft der Blätter der Aloe barbadensis und enthält neben Harzen, Anthranoiden, Emodin, ätherischen Ölen und dgl., 15-40% Aloin, ein bitter schmeckendes, gelbliches Anthron-Derivat, das als Abführmittel in der Bevölkerung sehr beliebt ist. Die Wirkung kommt durch Darmschleimhaut-Reizung zustande und setzt schon nach einigen Stunden ein. Andere Beschwerden werden mit Hilfe der Aloe Vera behandelt: Afterentzündung, Arteriosklerose, eiternde Wunden und Verletzungen, Wundrose, Zwölffingerdarmgeschwür, Faulecken, Haarausfall, Hämorrhoiden, Krampfadern, Pilzerkrankungen auf der Haut, zur Hautpflege und Blutreinigung ("Aloe Vera - Die Königin der Heilpflanzen" von Alice Beringer, S. 13-53, Hyene Bücher, 1997, Wilhelm Hyene Verlag, München). Im erfindungsgemäßen Stoffgemisch fördert Aloe Vera die feuchtigkeitsspendende und durchblutende Wirkung, was sich äußerst positiv auf die zu behandelnde Erkrankung auswirkt.

Die Inhaltsstoffe des Thymians (Thymus vulgaris) sind ätherische Öle, Gerbstoffe, Phenolcarbonsäuren, Kohlenhydrate, Triterpene, Aluminium und verschiedene Biphenylderivate. Thymian besitzt antimikrobielle, antivirale, insektizide, spasmolytische, wundheilende, anthelmintische und antioxidative Wirkung, weiters hemmt Thymian die Prostaglandinsynthese und beeinflusst den Arzneistoffmetabolismus ("Hagars Handbuch der pharmazeutischen Praxis" von R. Hänsel, K. Keller, H. Rimpler, G. Schneider, Band 6, Drogen P-Z, S. 974-989, 5. Auflage, 1994, Springer Verlag). Bevorzugterweise wird daher auch Thymian dem erfindungsgemäßen Stoffgemisch zugesetzt, wodurch die wundheilende und entzündungshemmende Wirkung sowie die Linderung von Juckreiz erhöht und verbessert wird.

Ein weiterer möglicher und wünschenswerter Zusatz zum erfindungsgemäßen Stoffgemisch ist der von Lavendel (Lavandula). Die Inhaltsstoffe des Lavendels sind unter anderem ätherische Öle, Gerbstoffe, Phenylcarbonsäuren. Unter den bedeutendsten Wirkungen sind chloretische und cholagoge, blutzuckersenkende, narbenglättende, schmerz- und krampflindernde, antimikrobielle und expektorierende zu nennen ("Hagars Handbuch der pharmazeutischen Praxis" von R. Hänsel, K. Keller, H. Rimpler, G. Schneider, Band 5, Drogen E-O, S. 621, 630, 634, 642, 5. Auflage, 1993). Durch den Zusatz von Lavendel wirkt das erfindungsgemäße Stoffgemisch zusätzlich gegen Verspannungen, schlecht heilende Wunden, chronische Ekzeme im Darm- und Analbereich sowie zur Anregung des Kreislaufs.

Weitere Öle, wie z.B. Johanneskraut, Mandelöl, Rosenöl oder Weizenöl vervollständigen die Inhaltsstoffe des Präparates.

Eine besonders günstige Wirkung des Stoffgemischs wird erreicht, wenn es Bienenwachs zu 10-30%, bevorzugterweise zu 18%, Propolis zu 1-15%, bevorzugterweise zu 5%, und/oder Kamille zu 1-10%, bevorzugterweise zu 2%, und/oder Salbei zu 1-10%, bevorzugterweise zu 2%, und/oder Aloe Vera zu 1-10%, bevorzugterweise zu 5,3% und/oder Thymian zu 0,1-10%, bevorzugterweise zu 1%, und/oder Lavendel zu 0,01-5%, bevorzugterweise zu 0,2% enthält. Dadurch werden die Eigenschaften dieser verschiedenen Stoffe optimal ausgenützt, weil sie aufeinander abgestimmt sind. Es können dabei einer, mehrere oder alle zugesetzt werden; besonders günstig ist aber ein Stoffgemisch, in dem alle in dem hier mit "bevorzugterweise" gekennzeichneten Anteil enthalten sind.

Eine vorteilhafte Variante des Stoffgemischs besteht auch darin, dass es weitere pflanzliche Substanzen enthält, wie z.B. Birkenöl, Bois Bondee Rinde, Gelee Royale, Jasmin, Johanneskrautöl, Majoran, Myrrhe, Mandelöl, Pfefferminze, Pollen, Rosenöl, Rosmarin, Sandelholz, Schafgarbe, Weizenöl, Zederholzöl, Zimt und/oder Zypresse.

Das Stoffgemisch ist aber nicht auf die oben genannten Inhaltsstoffe beschränkt, sondern kann durch zusätzliche Komponenten ergänzt werden, so z.B. weitere ätherische Öle, Vitamine, Fettsäuren, Mineralstoffe, Kohlenhydrate, organische und anorganische Säuren, und andere, die vorteilhafterweise dann zugemischt werden können, wenn es die spezifische Anwendung erfordert.

Eine günstige Ausführungsform des erfindungsgemäßen Stoffgemischs ist weiters gegeben, wenn das Gemisch zusätzlich homöopathische Substanzen enthält. Diese homöopathischen Substanzen können durch ihre besonderen Eigenschaften entweder die heilende Wirkung des erfindungsgemäßen Stoffgemischs verstärken, oder sie können eine zusätzliche Wirkung verursachen, je nachdem welches homöopathische Mittel zugesetzt wird.

Bevorzugt verwendet wird ein Stoffgemisch umfassend unabhängig voneinander Honig zu 30-80%, insbesondere zu 50-60%, Olivenöl zu 1-20%, insbesondere zu 7-10% und Bienenwachs zu 1-30%, insbesondere zu 15-20%.

Das erfindungsgemäß zu verwendende Mittel kann dabei in allen für topische Arzneimittel gebräuchlichen Applikationsformen bereitgestellt werden, insbesondere als Salbe, Emulsion, Schwamm, Seife, Badesalz, Lotion,...

Gemäß einem weiteren Aspekt betrifft die vorliegende Erfindung ein Stoffgemisch, welches Honig zu 50-90%, insbesondere zu 50-60%, Olivenöl zu 1-20%, insbesondere zu 7-10%, Bienenwachs zu 1-30%, insbesondere zu 15-20% und Aloe Vera enthält. Es hat sich herausgestellt, dass die positiven Eigenschaften der Salbe bestmöglichst herausgeholt werden, wenn der Honig eine Mischung aus Blütenhonig, Waldhonig und Akazienhonig ist. Optimal ist weiters ein Zusatz von etwa 8,5% Olivenöl, da das erfindungsgemäße Mittel hierbei die größte Effektivität bei Hämorrhoidenleiden zeigt.

Des Weiteren ist es günstig, wenn das Stoffgemisch zusätzlich eine, mehrere oder alle der folgenden Substanzen enthält: Propolis, Kamille, Salbei, Aloe Vera, Thymian, Lavendel und/oder diverse Öle.

Ein besonders vorteilhaftes Stoffgemisch wird erreicht, wenn es Propolis zu 1-15%, bevorzugterweise zu 5%, und/oder Kamille zu 1-10%, bevorzugterweise zu 2%, und/oder Salbei zu 1-10%, bevorzugterweise zu 2%, und/oder Aloe Vera zu 1-10%, bevorzugterweise zu 2%, und/oder Thymian zu 0,1-10%, bevorzugterweise zu 1%, und/oder Lavendel zu 0,01-5%, bevorzugterweise zu 0,2% enthält.

Weiters ist es vorteilhaft, wenn das Stoffgemisch zusätzliche Komponenten, wie weitere pflanzliche Substanzen (wie z.B. Birkenöl, Bois Bondee Rinde, Gelee Royale, Jasmin, Johanneskrautöl, Majoran, Myrrhe, Mandelöl, Pfefferminze, Pollen, Rosenöl, Rosmarin, Sandelholz, Schafgarbe, Weizenöl, Zederholzöl, Zimt und/oder Zypresse), homöopathische Substanzen oder Mischungen dieser Komponenten umfasst. Weiters kann das erfindungsgemäße Stoffgemisch auch Substanzen umfassen, die seine Anwendung bzw. Haltbarkeit unterstützen, wie z.B. Stabilisatoren, Konservierungsmittel, pharmazeutische Hilfsstoffe, Puffersubstanzen, Verdickungsmittel, Emulgatoren,...

Die Erfindung wird anhand des nachfolgenden Beispiels, auf das sie selbstverständlich nicht eingeschränkt ist, weiter erläutert werden.

### Beispiel:

### Behandlung von Hämorrhoiden-Patienten :

Zusammensetzung der Salbe :
Honig (55%), wobei 30% Blüten-, 30% Akazien- und 40% Waldhonig darin enthalten sind, Olivenöl (8,5%), Propolis (5% einer 40%-Tinktur), Bienenwachs (18%), Kamillentinktur, Salbeitinktur, Lavendel, Thymian, Aloe Vera, Weizenöl.

36 Patienten mit Hämorrhoiden (21 Frauen zwischen 23-64 Jahren und 15 Männer zwischen 25-66 Jahren) wurden mit folgender Applikationsvorschrift 4 Wochen lang behandelt, wobei die Salbe in der ersten Woche 3mal täglich und in den weiteren Wochen 2mal täglich appliziert wurde : Die Tubenverlängerung der Salbe wurde etwa 3-5 cm in das Rektum eingeführt, die Salbe nach Gefühl hineingedrückt, während die Tubenverlängerung langsam zurückgezogen wurde. Die Salbe wurde auch rund um den Anus aufgetragen, danach trocknen gelassen und eine beliebige Einlage verwendet (ohne abzuwischen). Nach etwa 20 min wurde die Einlage entfernt.

Kurze Zeit nach der Anwendung kam es in einigen Fällen zu leichtem Brennen, anschließend trat jedoch das Wohlgefühl der Schmerzlinderung und der juckreizhemmenden Wirkung bis zum Verschwinden der Beschwerden ein.

Bei der Mehrzahl der Probanden kam es am 2. Tag zu einem Blutungsstillstand bei weitgehender Beschwerdefreiheit. Bei 8 Patienten kam es am 6. Tag zu einem Blutungsstillstand und zu einer wesentlichen Besserung; diese Patienten litten allerdings bereits zwischen 2 und 11 Jahren an Hämorrhoiden.

16 der Testpersonen litten zusätzlich an perianalen Thrombosen; auch bei dieser Erkrankung zeigte sich unter Behandlung eine weitgehende Rückbildung. Bei 3 Patienten war aufgrund ihrer hämorrhoidalen Erkrankung eine Operation vorgesehen, deren Indikation durch einen Facharzt festgestellt worden war. Diese Indikation bestand nach der Behandlung nicht mehr (auch durch den Facharzt festgestellt).

## Patentansprüche

1. Verwendung eines Stoffgemischs, umfassend Honig und Olivenöl zur Herstellung eines Mittels zur Behandlung von hämorrhoidalen Erkrankungen.

2. Verwendung eines Stoffgemischs nach Anspruch 1, **dadurch gekennzeichnet, dass** das Gemisch Honig zu 50-90% enthält.

3. Verwendung eines Stoffgemischs nach Anspruch 1 oder 2, **dadurch gekennzeichnet, dass** das Gemisch Olivenöl zu 5-20%, vorzugsweise zu etwa 8,5%, enthält.

4. Verwendung eines Stoffgemischs nach einem der Ansprüche 1 bis 3, **dadurch gekennzeichnet, dass** das Gemisch zusätzlich eines, mehrere oder alle der folgenden Substanzen enthält: Bienenwachs, Propolis, Kamille, Salbei, Aloe Vera, Thymian, Lavendel und/oder diverse Öle.

5. Verwendung eines Stoffgemischs nach einem der Ansprüche 1 bis 4, **dadurch gekennzeichnet, dass** das Gemisch Bienenwachs zu 10-30% enthält.

6. Verwendung eines Stoffgemischs nach einem der Ansprüche 1 bis 5, **dadurch gekennzeichnet, dass** das Gemisch Propolis zu 1-15% enthält.

7. Verwendung eines Stoffgemischs nach einem der Ansprüche 1 bis 6, **dadurch gekennzeichnet, dass** das Gemisch Kamille zu 1-10% enthält.

8. Verwendung eines Stoffgemischs nach einem der Ansprüche 1 bis 7, **dadurch gekennzeichnet, dass** das Gemisch Salbei zu 1-10% enthält.

9. Verwendung eines Stoffgemischs nach einem der Ansprüche 1 bis 8, **dadurch gekennzeichnet, dass** das Gemisch Aloe Vera zu 1-10% enthält.

10. Verwendung eines Stoffgemischs nach einem der Ansprüche 1 bis 9, **dadurch gekennzeichnet, dass** das Gemisch Thymian zu 0,1-10% enthält.

11. Verwendung eines Stoffgemischs nach einem der Ansprüche 1 bis 10, **dadurch gekennzeichnet, dass** das Gemisch Lavendel zu 0,01-5% enthält.

12. Verwendung eines Stoffgemischs nach einem der Ansprüche 1 bis 11, **dadurch gekennzeichnet, dass** das Gemisch zusätzlich weitere pflanzliche Substanzen enthält.

13. Verwendung eines Stoffgemischs nach einem der Ansprüche 1 bis 12, **dadurch gekennzeichnet, dass** das Gemisch zusätzlich homöopathische Substanzen enthält.

14. Verwendung eines Stoffgemischs nach einem der Ansprüche 1 bis 13, **dadurch gekennzeichnet, dass** das Gemisch unabhängig voneinander Honig zu 30-80%, insbesondere zu 50-60%, Olivenöl zu 1-20%, insbesondere zu 7-10% und Bienenwachs zu 1-30%, insbesondere zu 15-20% umfasst.

15. Stoffgemisch, **dadurch gekennzeichnet, dass** es Honig zu 50-90%, insbesondere zu 50-60%, Olivenöl zu 1-20%, insbesondere zu 7-10%, Bienenwachs zu 1-30%, insbesondere zu 15-20% und Aloe Vera enthält.

16. Stoffgemisch nach Anspruch 15, **dadurch gekennzeichnet, dass** das Gemisch zusätzlich eines, mehrere oder alle der folgenden Substanzen enthält: Propolis, Kamille, Salbei, Aloe Vera, Thymian, Lavendel und/oder diverse Öle.

17. Stoffgemisch nach Anspruch 15 oder 16, **dadurch gekennzeichnet, dass** das Gemisch Propolis zu 1-15% enthält.

18. Stoffgemisch nach einem der Ansprüche 15 bis 17, **dadurch gekennzeichnet, dass** das Gemisch Kamille zu 1-10% enthält.

19. Stoffgemisch nach einem der Ansprüche 15 bis 18, **dadurch gekennzeichnet, dass** das Gemisch Salbei zu 1-10% enthält.

20. Stoffgemisch nach einem der Ansprüche 15 bis 19, **dadurch gekennzeichnet, dass** das Gemisch Aloe Vera zu 1-10% enthält.

21. Stoffgemisch nach einem der Ansprüche 15 bis 20, **dadurch gekennzeichnet, dass** das Gemisch Thymian zu 0,1-10% enthält.

22. Stoffgemisch nach einem der Ansprüche 15 bis 21, **dadurch gekennzeichnet, dass** das Gemisch Lavendel zu 0,01-5% enthält.

23. Stoffgemisch nach einem der Ansprüche 15 bis 22, **dadurch gekennzeichnet, dass** das Gemisch zusätzlich weitere pflanzliche Substanzen enthält.

24. Stoffgemisch nach einem der Ansprüche 15 bis 23, **dadurch gekennzeichnet, dass** das Gemisch zusätzlich homöopathische Substanzen enthält.

## Claims

1. The use of a substance mixture comprising honey and olive oil for preparing a medicament for topical application.

2. The use of a substance mixture according to claim 1, **characterised in that** the mixture contains 50-90% of honey.

3. The use of a substance mixture according to claim 1 or 2, **characterised in that** the mixture contains 5-10% of olive oil.

4. The use of a substance mixture according to any one of claims 1 to 3, **characterised in that** the mixture additionally comprises one, several or all of the following substances: beeswax, propolis, camomile, sage, Aloe vera, thyme, lavender and/or diverse oils.

5. The use of a substance mixture according to any one of claims 1 to 4, **characterised in that** the mixture contains 10-30% of beeswax.

6. The use of a substance mixture according to any one of claims 1 to 5, **characterised in that** the mixture contains 1-15% of propolis.

7. The use of a substance mixture according to any one of claims 1 to 6, **characterised in that** the mixture contains 1-10% of camomile.

8. The use of a substance mixture according to any one of claims 1 to 7, **characterised in that** the mixture contains 1-10% of sage.

9. The use of a substance mixture according to any one of claims 1 to 8, **characterised in that** the mixture contains 1-10% of Aloe vera.

10. The use of a substance mixture according to any one of claims 1 to 9, **characterised in that** the mixture contains 0.1-10% of thyme.

11. The use of a substance mixture according to any one of claims 1 to 10, **characterised in that** the mixture contains 0.01-5% of lavender.

12. The use of a substance mixture according to any one of claims 1 to 11, **characterised in that** the mixture additionally contains further vegetable substances.

13. The use of a substance mixture according to any one of claims 1 to 12, **characterised in that** the mixture additionally contains homeopathic substances.

14. The use of a substance mixture according to any one of claims 1 to 13 for preparing a remedy for treating hemorrhoidal diseases.

15. The use of a substance mixture according to claim 14, **characterised in that** the mixture independently comprises 30-80%, in particular 50-60%, of honey, 1-20%, in particular 7-10%, of olive oil, and 1-30%, in particular 15-20%, of beeswax.

16. The use of a substance mixture according to any one of claims 1 to 13 for preparing an agent for hair and scalp care, preferably against loss of hair, dandruff formation, dehydration.

17. The use of a substance mixture according to claim 16, **characterised in that** the mixture independently comprises 10-40%, in particular 20-30% of honey, 1-10%, in particular 4-6% of olive oil, and 1-40%, in particular 15-25% of Aloe vera.

18. The use of a substance mixture according to any one of claims 1 to 13 for preparing an agent for skin care.

19. The use of a substance mixture according to claim 18, **characterised in that** the mixture independently comprises 1-40%, in particular 15-25%, of honey, 1-30%, in particular 10-20% of olive oil, and 10-50%, in particular 20-40%, of Aloe vera.

20. The use of a substance mixture according to any one of claims 1 to 13 for preparing a remedy for varicose veins.

21. A substance mixture, **characterised in that** it comprises 50-90% of honey, and olive oil.

22. A substance mixture according to claim 21, **characterised in that** the mixture comprises 5-15% of olive oil.

23. A substance mixture according to claim 21 or 22, **characterised in that** the mixture additionally comprises one, several or all of the following substances: beeswax, propolis, camomile, sage, Aloe vera, thyme, lavender and/or various oils.

24. A substance mixture according to any one of claims 21 to 23, **characterised in that** the mixture contains 10-30% of beeswax.

## Revendications

1. Utilisation d'un mélange de substances contenant du miel et de l'huile d'olive destiné à la fabrication d'un médicament pour usage topique, la proportion de miel dans le mélange devant atteindre 20%.

2. Utilisation d'un mélange de substances conformément au droit n° 1, **caractérisé par le fait que** le mélange contient 50-90 % de miel

3. Utilisation d'un mélange de substances conformément à l'un des droits 1 ou 2, **caractérisé par le fait que** le mélange contient de l'huile d'olive à hauteur de 1-30%, plus particulièrement 4-15%

4. Utilisation d'un mélange de substances conformément à un des droits 1 à 3, **caractérisé par le fait que** le mélange contient en outre une , plusieurs ou toutes les substances suivantes : cire d'abeille, propolis, camomille, sauge, Aloe vera, Thym, Lavande et/ou diverses huiles

5. Utilisation d'un mélange de substances conformément à l'un des droits 1 à 4, **caractérisé par le fait que** le mélange contient de la cire d'abeille à hauteur de 1-30 %.

6. Utilisation d'un mélange de substances conformément à l'un droits 1 à 5, **caractérisé par le fait que** le mélange contient de la propolis à hauteur de 1-15% ( d'une teinture à 40%).

7. Utilisation d'un mélange de substances conformément à l'un des droits 1 à 6, **caractérisé par le fait que** le mélange contient de la camomille à hauteur de 1-10%

8. Utilisation d'un mélange de substances conformément à l'un des droits 1 à 7, **caractérisé par le fait que** le mélange contient de la sauge à hauteur de 1-10%

9. Utilisation d'un mélange de substances conformément à l'un des droits 1 à 8, **caractérisé par le fait que** le mélange contient de l'Aloe vera à hauteur de 1-50%, plus particulièrement 20-30%

10. Utilisation d'un mélange de substances conformément à l'un des droits 1 à 9, **caractérisé par le fait que** le mélange contient du thym à hauteur de 0,1-10%

11. Utilisation d'un mélange de substances conformément à l'un des droits 1 à 10, **caractérisé par le fait que** le mélange contient de la lavande à hauteur de 0,01-5%

12. Utilisation d'un mélange de substances conformément à l'un des droits 1 à 11, **caractérisé par le fait que** le mélange contient en outre d'autres substances végétales

13. Utilisation d'un mélange de substances conformément à l'un des droits 1 à 12, **caractérisé par le fait que** le mélange contient en outre des substances homéopathiques

14. Utilisation d'un mélange de substances conformément à l'un des droits 1 à 13, destiné à la fabrication d'un remède destiné au traitement des maladies hémorroïdales

15. Utilisation d'un mélange de substances conformément au droit 15, **caractérisé par le fait que** le mélange contient, indépendamment les uns des autres, du miel à hauteur de 30-80%, plus particulièrement 50-60%, de l'huile d'olive à hauteur de 1-20%, plus particulièrement 7-10%, de la propolis à hauteur de 1-10% (d'une teinture à 40%), plus particulièrement 3-7% (d'une teinture à 40%) et de la cire d'abeille à hauteur de 1-30%, plus particulièrement 15-20%

16. Utilisation d'un mélange de substances conformément à l'un des droits 1 à 13, destiné à la fabrication d'une préparation pour les soins des cheveux et du cuir chevelu, plus spécialement indiqué en cas de chute de cheveux, formation de pellicules et assèchement.

17. Utilisation d'un mélange de substances conformément au droit 16, **caractérisé par le fait que** le mélange contient, indépendamment les uns des autres, du miel à hauteur de 20-40 %, plus particulièrement 25-30%, de l'huile d'olive à hauteur de 1-10%, plus particulièrement 4-6%, de la propolis à hauteur de 1-10% (d'une teinture à 40%), plus particulièrement 2-4% (d'une teinture de 40%) et de l'Aloe vera à hauteur de 1-40%, plus particulièrement 15-25%.

18. Utilisation d'un mélange de substances conformément à l'un des droits 1 à 13 destiné à la fabrication d'une préparation pour les soins de la peau

19. Utilisation d'un mélange de substances conformément au droit 18, **caractérisé par le fait que** le mélange contient indépendamment les uns des autres, du miel à hauteur de 20-40 %, plus particulièrement 20-25%, de l'huile d'olive à hauteur de 1-30%, plus particulièrement 10-20%, de la propolis à hauteur de 1-15% (d'une teinture à 40%), plus particulièrement 5-10% (d'une teinture de 40%) et de l'Aloe vera à hauteur de 10-50%, plus particulièrement 20-40%.

20. Utilisation d'un mélange de substances conformément à l'un des droits 1 à 13, destiné à la fabrication d'un remède contre les varices

21. Mélange de substances **caractérisé par le fait que** le mélange contient du miel à hauteur minimum de 20% et de l'huile d'olive

22. Mélange de substances conformément au droit 21, **caractérisé par le fait que** le mélange contient 50-90 % de miel

23. Mélange de substances conformément au droit 21 ou 22, **caractérisé par le fait que** le mélange contient de l'huile d'olive à hauteur de 1-30%, plus particulièrement 4-15%

24. Mélange de substances conformément à l'un des droits 21 à 23, **caractérisé par le fait que** le mélange contient en outre une, plusieurs ou toutes les substances suivantes : cire d'abeille, propolis, camomille, sauge, Aloe vera, thym, lavande et/ou diverses huiles.
